(19) Europäisches Patentamt
European Patent Office
Office européen des brevets

(11) Veröffentlichungsnummer: **0 022 942**
**A1**

(12) **EUROPÄISCHE PATENTANMELDUNG**

(21) Anmeldenummer: **80103636.9**

(22) Anmeldetag: **27.06.80**

(51) Int. Cl.[3]: **C 07 C 113/04**
C 07 F 5/02, C 07 C 17/22
C 07 C 25/13

(30) Priorität: **11.07.79 DE 2927938**

(43) Veröffentlichungstag der Anmeldung:
**28.01.81 Patentblatt 81/4**

(84) Benannte Vertragsstaaten:
**BE CH DE FR GB IT LI NL**

(71) Anmelder: **BAYER AG**
**Zentralbereich Patente, Marken und Lizenzen**
**D-5090 Leverkusen 1, Bayerwerk(DE)**

(72) Erfinder: **Hagemann, Hermann, Dr.**
**Roggendorfstrasse 55**
**D-5000 Koeln 80(DE)**

(72) Erfinder: **Baasner, Bernd, Dr.**
**Kalkstrasse 132**
**D-5090 Leverkusen 1(DE)**

(72) Erfinder: **Klauke, Erich, Dr.**
**Eichendorffweg 8**
**D-5068 Odenthal(DE)**

(54) **Verfahren zur Herstellung von Diazoniumtetrafluoroboraten mittels Nitrosyltetrafluoroborat und deren Verwendung.**

(57) Es wurde ein Verfahren zur Herstellung von Diazoniumtetrafluoroboraten der Formel (I) gefunden,

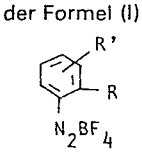

1

das dadurch gekennzeichnet ist, daß man Aniline der Formel (II) oder deren Säureadditionssalze

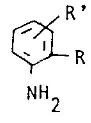

11

mit Nitrosyltetrafluoroborat in Anwesenheit eines inerten organischen Verdunnungsmittels umsetzt.

EP 0 022 942 A1

Croydon Printing Company Ltd.

BEZEICHNUNG GEÄNDERT
siehe Titelseite



BAYER AKTIENGESELLSCHAFT
Zentralbereich
Patente, Marken und Lizenzen

5090 Leverkusen, Bayerwerk
Rt/Kü-c
Typ IVa/ZP

# Verfahren zur Herstellung von Diazoniumtetrafluoroboraten mittels Nitrosyltetrafluoroborat

Die vorliegende Erfindung betrifft ein Verfahren zur Herstellung von Diazoniumtetrafluoroboraten mittels Nitrosyltetrafluoroborat.

3-Brom-4-fluortoluol wird erhalten durch Zersetzung von 2-Brom-4-methylphenyldiazoniumtetrafluoroborat. Es ist bekannt, daß man das 2-Brom-4-methylphenyldiazoniumtetrafluoroborat durch Diazotierung des 3-Brom-4-aminotoluols bzw. dessen Hydrochlorids mit Natriumnitrit in wäßriger Tetrafluorborsäure-Lösung herstellen kann. Jedoch beträgt die Ausbeute lediglich 73,5 %. Wegen ungenügender Reinheit des so hergestellten Tetrafluoroborats werden bei der anschließenden Zersetzung nur 43 % an 3-Brom-4-fluortoluol erhalten (J.M.W. Scott, Can. J. Chem. 38, 2444 (1960)).

Es ist außerdem bekannt, daß man Nitrosyltetrafluoroborat in nichtwäßrigem Medium als Diazotierungsmittel und Tetrafluoroborationenquelle verwenden kann, jedoch wurde dieses Verfahren bislang lediglich in Tetrachlorkohlenstoff als Lösungsmittel und unsubstituiertem

Anilin als Substrat in guten Ausbeuten angewandt (U. Wannagat u. G. Hohlstein. Chem. Ber. 88, 1839 (1955)).

Es wurde nun ein Verfahren zur Herstellung von Diazoniumtetrafluoroboraten der Formel (I) gefunden,

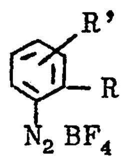

I

in welcher
R und R' für Alkyl, Halogen, $SCF_3$, $OCF_3$ stehen,

dadurch gekennzeichnet, daß man Aniline der Formel (II) oder deren Säureadditionssalze,

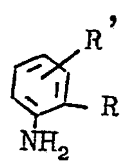

II

in welcher R und R' die oben angegebene Bedeutung haben,

mit Nitrosyltetrafluoroborat in Anwesenheit eines inerten organischen Verdünnungsmittels umsetzt.

Nach diesem Verfahren werden in überraschend hoher Ausbeute (92 %) und in hervorragender Reinheit die Diazoniumtetrafluoroborate erhalten. Dies war um so überraschender, als aufgrund der sterischen Verhältnisse

(Substituent in ortho-Stellung zur $NH_2$-Gruppe) eine Hinderung der Reaktion hätte erwartet werden müssen.

Verwendet man 3-Brom-4-aminotoluol und Nitrosyltetrafluoroborat als Ausgangsstoffe, so kann der Reaktionsverlauf durch das folgende Formelschema wiedergegeben werden:

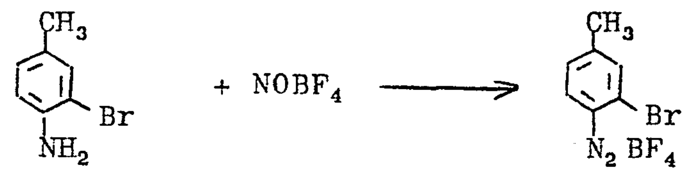

Als Ausgangsamine werden bevorzugt Aniline der Formel II oder deren Säureadditionsverbindungen eingesetzt, bei denen R und/oder R' Methyl, Ethyl, Brom, Chlor, Fluor, $SCF_3$ und $OCF_3$ bedeuten.

Die erfindungsgemäß verwendbaren Aniline sind bekannt. Als Beispiele seien 3-Brom-4-aminotoluol und 6-Chlor-2-aminotoluol genannt.

Das Nitrosyltetrafluoroborat wird in Ausbeuten von 95 bis 98 % aus äquimolaren Mengen Fluorwasserstoff, Bortrifluorid und einem Nitrosierungsmittel (wie z.B. Nitrosylchlorid) in einem polaren, inerten anorganischen

oder organischen Lösungsmittel, wie z.B. Schwefeldioxid oder Nitromethan, synthetisiert (Gmelin, Handbuch der anorg. Chemie, Ergänzungswerk zur 8. Auflage Bd. 37 Teil 10, S. 246):

$$BF_3 + NOCl + HF \longrightarrow NOBF_4$$

Die Synthese der Diazoniumtetrafluoroborate wird in organischen oder anorganischen Lösungsmitteln duchgeführt. Hierzu gehören vorzugsweise halogenierte Kohlenwasserstoffe wie Methylenchlorid, Chloroform, Tetrachlorkohlenstoff und Schwefeldioxid. Besonders bevorzugt ist die Duchführung der Reaktion in Methylenchlorid. Die Umsetzung wird bei Temperaturen zwischen -20°C und +80°C, vorzugsweise zwischen -5°C und +30°C vorgenommen.

Bei der Durchführung des erfindungsgemäßen Verfahrens setzt man auf 1 Mol Anilin 1 bis 2 Mol Nitrosyltetrafluoroborat, vorzugsweise 1 bis 1,2 Mol, ein. Dabei wird das Anilin z.B. als 20 bis 30 %ige Methylenchloridlösung in eine Suspension des Nitrosyltetrafluoroborats in Methylenchlorid eingetropft. Nach kurzer Zeit entstehen die Diazoniumtetrafluoroborate als kristalline Niederschläge, die zweckmäßigerweise abgesaugt und zur Entfernung des entstandenen Reaktionswassers mit Alkoholen (vorzugsweise Ethanol) und einem unpolaren Kohlenwasserstoff oder Kohlenwassertoffgemisch gewaschen

und anschließend in der Wärme getrocknet werden können. Es ist auch möglich, die abgetrennten Diazoniumtetrafluoroborate in Methanol zu lösen und mit Ether oder einem Kohlenwasserstoff auszufällen.
Das bei der beschriebenen Reaktions entstehende Wasser kann auch durch Zusatz von Thionylchlorid zur Reaktionsmischung gebunden werden.

Die so erhaltenen Diazoniumsalze können ohne weitere Reinigung zur nachfolgenden Zersetzung verwendet werden, die durch das folgende Reaktionsschema erläutert wird:

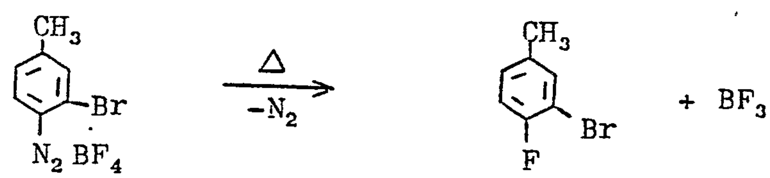


Zur Durchführung der Reaktion können die Diazoniumtetrafluoroborate in Substanz bis zu ihrer Zersetzungstemperatur erhitzt werden. Zur Verfahrensdurchführung (wegen besserer Dosierbarkeit und Steuerung der Temperatur der Zersetzungsmasse) ist es jedoch wesentlich günstiger, die Zersetzung in Gegenwart eines geeigneten Verdünnungsmittels durchzuführen. Beschrieben ist, daß die Zersetzung in Anwesenheit von Sand durchführbar ist, jedoch schließt sich eine aufwendige Abtrennung

des Reaktionsproduktes an ( s. z.B. J.M.W. Scott, Can.J.Chem. 38 (1960), 2444). Es wurde nunmehr gefunden, daß die Zersetzung auch in inerten organischen Lösungsmitteln, am vorteilhaftesten jedoch in dem erwünschten Reaktionsprodukt selbst als Verdünnungsmittel, vorgenommen werden kann.
Dazu werden die festen Diazoniumtetrafluoroborate mit Hilfe geeigneter Einrichtungen (z.B. einer Schnecke) oder als Suspension mit einer Dosierpumpe kontinuierlich in das auf die jeweilige Diazoniumsalz-Zersetzungstemperatur gebrachte Reaktionsgefäß eingebracht, in dem als Verdünnungsmittel von dem jeweils zu synthetisierenden kernfluorierten Aromaten vorgelegt ist.

Das molare Verhältnis von zu zersetzendem Diazoniumtetrafluoroborat zu Verdünnungsmittel beträgt 1 zu 0,2 bis 2, am vorteilhaftesten 1 zu 0,4 bis 0,8.
Aus dem Zersetzungsrückstand wird das gewünschte Produkt mit Hilfe bekannter Methoden (Destillation, Kristallisation) abgetrennt. Die Ausbeuten bei diesen Zersetzungsreaktionen betragen bis zu 95 %.

Das bei der Zersetzungsreaktion abgespaltene Bortrifluorid wird in der oben bereits erläuterten Weise zur erneuten Synthese des Nitrosyltetrafluoroborats eingesetzt.

Die erhaltenen kernfluorierten Aromaten sind wertvolle Zwischenprodukte zur Herstellung von insektiziden Wirkstoffen.

Beispiele

2-Brom-4-methylphenyldiazoniumtetrafluoroborat

Zu 245 g (2,1 Mol) Nitrosyltetrafluoroborat, die in 1000 ml Methylenchlorid suspendiert sind, tropft man bei +5° bis +10°C innerhalb von 90 min eine Lösung von 372 g (2 Mol) 3-Brom-4-aminotoluol in 1400 ml $CH_2Cl_2$ ein. Man läßt unter Rühren auf Raumtemperatur kommen, saugt den Niederschlag ab, wäscht mit wenig Ethanol, anschließend mit Petrolether und trocknet bei 50°C. Die Ausbeuten betragen 500 bis 525 g (88 - 92 %), Fp. (Zers.): 124 - 6°C.

Zersetzung des 2-Brom-4-methylphenyldiazoniumtetrafluoroborats zum 3-Brom-4-fluortoluol und Wiedergewinnung des Bortrifluorids.

285 g (1 Mol) 2-Brom-4-methylphenyldiazoniumtetrafluoroborat werden mit Hilfe einer Schnecke während 90 min in ein Reaktionsgefäß dosiert, in dem 100 ml 3-Brom-4-fluortoluol auf 100° bis 110°C erwärmt wurden. Das abgespaltene Gasgemisch wird über eine Kolonne in einen unter -10°C gekühlten Stahlautoklaven eingeleitet, der mit 300 ml Schwefeldioxid, in dem 20 g HF und 65 g NOCl gelöst sind, beschickt ist.
Nach beendeter Zersetzung wird das Schwefeldioxid abdestilliert, der kristalline Rückstand kurz im Wasserstrahlvakuum getrocknet. Das ausgefallene Nitrosyltetrafluoroborat (102 g, 87 % Ausbeute) kann ohne weitere Reinigung erneut zur Synthese von Diazoniumtetrafluoroboraten eingesetzt werden.

Der angefallene Zersetzungsrückstand wird im Wasserstrahlvakuum destilliert und liefert 170 g (90 %) an 3-Brom-4-fluortoluol vom Kp. 72 - 3°C (18 Torr).

Patentansprüche:

1. Verfahren zur Herstellung von Diazoniumtetrafluoroboraten der Formel (I),

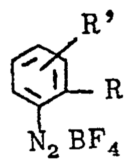

I.

in welcher R und R' für Alkyl, Halogen, $SCF_3$, $OCF_3$ stehen,

dadurch gekennzeichnet, daß man Aniline der Formel (II) oder deren Säureadditionssalze,

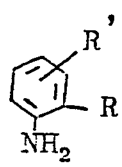

II

in welcher R und R' die oben angegebene Bedeutung haben,

mit Nitrosyltetrafluoroborat in Anwesenheit eines inerten organischen Verdünnungsmittels umsetzt.

2. Verwendung der nach dem Verfahren gemäß Anspruch 1 erhaltenen Tetrafluoroborate zur Herstellung der entsprechenden Fluorverbindungen, dadurch gekennzeichnet, daß man die Tetrafluoroborate erhitzt.

3. Verwendung der nach dem Verfahren gemäß Anspruch 1 erhaltenen Tetrafluoroborate gemäß Anspruch 2, dadurch gekennzeichnet, daß die Tetrafluoroborate in Anwesenheit der entsprechenden Fluorverbindungen als Verdünnungsmittel erhitzt werden.

0022942

Europäisches Patentamt

# EUROPÄISCHER RECHERCHENBERICHT

Nummer der Anmeldung: EP 80 10 3636

| EINSCHLÄGIGE DOKUMENTE | | | KLASSIFIKATION DER ANMELDUNG (Int Cl.) |
|---|---|---|---|
| Kategorie | Kennzeichnung des Dokuments mit Angabe, soweit erforderlich, der maßgeblichen Teile | betrifft Anspruch | |
| | CHEMICAL ABSTRACTS, Band 32, Nr.21, 10.November 1938, Spalte 8379[5] Columbus, Ohio. USA S.A. VOZNESENSKII et al."Nitrosyl borofluoride and its use in the fluorination".<br>& J. Gen. Chem. (USSR) 8, 524-8 (in French 528) (1938).<br>* Ganze Zusammenfassung *<br>-- | 1-3 | C 07 C 113/04<br>C 07 F 5/02<br>C 07 C 17/22<br>25/13 |
| D | CHEMISCHE BERICHTE 88.Jahrgang, Nr. 12, Seiten 1839-1846 (1955) U. WANNAGAT et al. "Zur Fluorierung mit Nitrosyl-tetrafluoroborat"<br>* Seite 1843, Absatz 4*<br>-- | 1 | RECHERCHIERTE SACHGEBIETE (Int. Cl.)<br>C 07 C 113/04<br>C 07 F 5/02<br>C 07 C 25/13 |
| | GB - A - 833 163 (DOW CHEMICAL CO.)<br>* Seite 2, Zeilen 95-125 *<br>---- | 2,3 | |

KATEGORIE DER GENANNTEN DOKUMENTE
- X: von besonderer Bedeutung
- A: technologischer Hintergrund
- O: nichtschriftliche Offenbarung
- P: Zwischenliteratur
- T: der Erfindung zugrunde liegende Theorien oder Grundsätze
- E: kollidierende Anmeldung
- D: in der Anmeldung angeführtes Dokument
- L: aus andern Gründen angeführtes Dokument
- &: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 20-10-1980 | SUTER |